# EUROPEAN PATENT APPLICATION

(11) **EP 4 592 679 A1**
(43) Date of publication of application: **30.07.2025**
(21) Application number: 23867417.0
(22) Date of filing: 15.09.2023
(51) Int. Cl.: G01N 33/573, C07K 16/40, G16H 50/20

(54) **USE OF DISCOIDIN DOMAIN RECEPTOR 2 IN DIAGNOSIS OF NEURODEGENERATIVE DISEASES, AND RELATED COMPUTER READABLE MEDIUM**

(30) Priority: 20.09.2022 CN 202211142267
(71) Applicant: Fibroinova Biomedical Technology (Guangzhou) Company, Ltd., Guangzhou, Guangdong 510700 (CN)
(72) Inventor: SU, Jin, Guangzhou, Guangdong 510700 (CN); LI, Jia, Guangzhou, Guangdong 510700 (CN); JIANG, Haishan, Guangzhou, Guangdong 510700 (CN); WEI, Xinru, Guangzhou, Guangdong 510700 (CN)
(74) Representative: Patent 42
(86) International application number: PCT/CN2023/119127
(87) International publication number: WO 2024/061128

(57) **Abstract**

The present invention discloses use of an agent for detecting the expression level of discoidin domain receptor 2 (DDR2) in the preparation of a kit for diagnosing a neurodegenerative disease in a subject, wherein the level of DDR2 in a sample from the subject being higher than the level of a control not having the disease indicates that the subject has the neurodegenerative disease. The present invention also discloses a kit and a method for diagnosing a neurodegenerative disease, and a computer-readable storage medium. The present invention can efficiently and accurately diagnose the neurodegenerative disease by detecting DDR2.

## Description

### TECHNICAL FIELD

The present invention relates to a kit and a method for diagnosing a neurodegenerative disease, and a computer-readable medium, and more particularly, to diagnosing a neurodegenerative disease by detecting discoidin domain receptor 2 (DDR2).

### BACKGROUND

Neurodegenerative diseases are diseases in which various symptoms are caused by degenerative changes of nerve cells in the central nervous system, such as impaired motor and sensory functions, and inhibition of higher functions such as memory, learning, and computational reasoning. Examples of neurodegenerative diseases include, but are not limited to, Alzheimer's disease, Parkinson's disease, Huntington's disease, amyotrophic lateral sclerosis, frontotemporal dementia, spinocerebellar ataxia, brain trauma, gene mutations that secondarily affect the functions of cells in the central nervous system, etc. Neurodegenerative diseases have long been recognized as incurable complex diseases, and there are currently no effective drugs that improve the overall condition of neurodegenerative diseases by targeting systemic pathological processes.

The course of neurodegenerative diseases is usually an irreversible process. Therefore, the key to treatment is early diagnosis, intervention at the early stage of the disease, and delay of the progression of the disease course. If a rapid diagnosis can be carried out at the early stage of the disease onset of a patient or a potential patient can find the risk in advance and receive targeted treatment or prevention, it will help keep the disease course of the patient at the stage of slight damage and slow down the deterioration, thereby ensuring the quality of life of the patient and reducing the social burden.

At present, the diagnostic methods for various neurodegenerative diseases mainly include combined diagnosis, which mainly includes: neuropsychological assessment, cognitive impairment testing; brain imaging (e.g., brain PET scan or magnetic resonance imaging); cerebrospinal fluid marker detection; blood testing and genetic risk analysis, etc. However, the commonly used diagnostic methods either require injecting a certain dose of radioactive substances into a subject, or they involve significant invasive procedures that are prone to causing surgical infections. Therefore, the development of new markers for early diagnosis of neurodegenerative diseases is one of the important directions for future diagnosis and treatment.

Discoidin domain receptor 2 is a receptor tyrosine kinase (RTK) that utilizes the extracellular matrix protein collagen as its ligand. In addition to its kinase function, DDR2 promotes cell adhesion by activating β1-integrin. DDR2 has the unique functions of mediating the transmission of signals from the extracellular matrix into the cell, balancing the regulation of the extracellular matrix, and participating in the regulation of cell growth, differentiation, and metabolism. Although activation of DDR2 by extracellular matrix collagen is essential for normal development and tissue homeostasis, abnormal activation of these receptors following damage or disease is detrimental.

DDR2 is recognized as an important target for inflammation such as arthritis (e.g., osteoarthritis or rheumatoid arthritis) and fibrosis (e.g., pulmonary fibrosis, cirrhosis, renal fibrosis, or dermal fibrosis). DDR2 is mainly expressed in interstitial cells such as fibroblasts, myofibroblasts, and smooth muscle cells in the kidney, skin, lung, heart, and connective tissue. There is a lot of evidence that abnormal expression of DDR2 is related to a variety of disease processes, such as inflammation, hepatic fibrosis, renal fibrosis, pulmonary fibrosis, dermal scarring, and atherosclerosis. In the study of inflammation mouse model, it was found that DDR2 expression was upregulated in knee joints of aged mice. In study of rheumatoid arthritis rat model, it was found that DDR2 expression was upregulated in synovial cells of the rats.

However, the expression and function of DDR2 in nervous system cells, especially glial cells, have never been reported. At present, there is no research or report on the use of DDR2 in the diagnosis of neurodegenerative diseases.

### SUMMARY

The inventors of the present application have surprisingly found that the expression of DDR2 in a related sample was significantly increased in neurodegenerative disease patients compared with a normal control. The inventors have utilized an agent capable of binding to DDR2 to detect the presence and/or level of DDR2 in a sample, thereby efficiently and accurately diagnosing a neurodegenerative disease.

In a first aspect, the present invention provides a kit for diagnosing a neurodegenerative disease in a subject, the kit comprising an agent for detecting the expression level of discoidin domain receptor 2 (DDR2), wherein the level of DDR2 in a sample from the subject being higher than the level of a control not having the disease indicates that the subject has the neurodegenerative disease.

In a second aspect, the present invention provides use of an agent for detecting the expression level of discoidin domain receptor 2 (DDR2) in the preparation of a kit for diagnosing a neurodegenerative disease in a subject, wherein the level of DDR2 in a sample from the subject being higher than the level of a control not having the disease indicates that the subject has the neurodegenerative disease.

In a third aspect, the present invention provides an agent for detecting the expression level of discoidin domain receptor 2 (DDR2) for use in diagnosing a neurodegenerative disease in a subject, wherein the level of DDR2 in a sample from the subject being higher than the level of a control not having the disease indicates that the subject has the neurodegenerative disease.

In a fourth aspect, the present invention provides a method for diagnosing a neurodegenerative disease in a subject, the method comprising detecting the presence and/or level of discoidin domain receptor 2 (DDR2) in a sample from the subject. In some embodiments, the method comprises bringing an agent capable of binding to DDR2 into contact with a sample from the subject; detecting the presence of a complex formed by the agent and DDR2 in the sample after the contact; and determining that the subject has or is at risk of having a neurodegenerative disease based on the presence and/or level of the complex.

In a fifth aspect, the present invention provides a computer-readable storage medium having stored thereon computer instructions for reading and execution by a computer, the computer instructions being executed to perform a method for diagnosing whether a subject has a neurodegenerative disease, the method comprising: (a) bringing a sample from the subject into contact with an agent capable of binding to discoidin domain receptor 2 (DDR2); (b) detecting and reading a signal of the sample after the contact to determine whether the agent forms a complex with DDR2 in the sample; and (c) determining whether the signal exceeds a predetermined threshold, and when the signal exceeds the predetermined threshold, determining that the subject has the neurodegenerative disease, and wherein the threshold is a median level from subjects not having the disease.

In a sixth aspect, the present invention provides a method for treating a neurodegenerative disease in a subject, the method comprising: (a) bringing a sample from the subject into contact with an agent capable of binding to discoidin domain receptor 2 (DDR2); (b) detecting and reading a signal of the sample after the contact to determine whether the agent forms a complex with DDR2 in the sample; (c) determining whether the signal exceeds a predetermined threshold, and when the signal exceeds the predetermined threshold, determining that the subject has the neurodegenerative disease, and wherein the threshold is a median level from subjects not having the disease; and (d) administering a neuroprotective or neurorestorative therapy to the subject determined to have the neurodegenerative disease.

In any one of the aspects described above, the agent for detecting the expression level of DDR2 comprises an agent capable of binding to DDR2 to detect the level of DDR2 in the sample. In some embodiments, the agent capable of binding to DDR2 comprises a protein, a nucleic acid, or a small molecule compound. In some embodiments, the agent capable of binding to DDR2 is an anti-DDR2 monoclonal antibody or an antigen-binding fragment thereof, or an anti-DDR2 polyclonal antibody. In some embodiments, the anti-DDR2 monoclonal antibody is an anti-DDR2 nanobody.

In some embodiments, the agent capable of binding to DDR2 is labeled with a detectable label. In some embodiments, the detectable label is selected from a fluorescent label, a chemiluminescent label, a paramagnetic label, a radioisotope label, and an enzyme label.

In any one of the aspects described above, the sample is brain tissue or cerebrospinal fluid. In any one of the aspects described above, the neurodegenerative disease is a neurodegenerative disease mediated by damage to astrocytes. In any one of the aspects described above, the neurodegenerative disease is a neurodegenerative disease mediated by astrocytes with increased expression of DDR2, wherein the increased expression of DDR2 is determined by comparison with the expression level of DDR2 in astrocytes of a normal subject. Preferably, in any one of the aspects described above, the neurodegenerative disease is selected from Alzheimer's Disease (AD), amyotrophic lateral sclerosis (ALS), Parkinson's disease, Huntington's disease, frontotemporal dementia, spinal muscular atrophy, prion disease, spinocerebella axia, Friedreichs ataxia, primary lateral sclerosis, spinocerebellar atrophy, Machado-hoseph's disease, Lewy Body dementia, progressive bulbar palsy, progressive supranuclear palsy, and multiple system atrophy. In a more preferred embodiment, the neurodegenerative disease is Alzheimer's disease. In a more preferred embodiment, the neurodegenerative disease is amyotrophic lateral sclerosis.

In a seventh aspect, the present invention provides a kit for diagnosing a neurodegenerative disease in a subject, the kit comprising an exosome from the subject, and an agent capable of binding to discoidin domain receptor 2 (DDR2) to detect the level of DDR2 expressed by the exosome, wherein the level of DDR2 expressed by the exosome being higher than the level of a control not having the disease indicates that the subject has the neurodegenerative disease.

In an eighth aspect, the present invention provides use of an exosome from a subject in the preparation of a kit for diagnosing a neurodegenerative disease in the subject, wherein the level of discoidin domain receptor 2 (DDR2) expressed by the exosome being higher than the level of a control not having the disease indicates that the subject has the neurodegenerative disease.

In a ninth aspect, the present invention provides an exosome from a subject for use in diagnosing a neurodegenerative disease in the subject, wherein the level of discoidin domain receptor 2 (DDR2) expressed by the exosome being higher than the level of a control not having the disease indicates that the subject has the neurodegenerative disease.

In a tenth aspect, the present invention provides a method for diagnosing a neurodegenerative disease in a subject, the method comprising isolating an exosome from the subject and detecting the presence and/or level of discoidin domain receptor 2 (DDR2) in the exosome from the subject. In some embodiments, the method comprises bringing an agent capable of binding to DDR2 into contact with an exosome from the subject; detecting the presence of a complex formed by the agent and DDR2 in the exosome after the contact; and determining that the subject has or is at risk of having a neurodegenerative disease based on the presence and/or level of the complex.

In an eleventh aspect, the present invention provides a computer-readable storage medium having stored thereon computer instructions for reading and execution by a computer, the computer instructions being executed to perform a method for diagnosing whether a subject has a neurodegenerative disease, the method comprising: (a) isolating an exosome from the subject; (b) bringing the isolated exosome into contact with an agent capable of binding to discoidin domain receptor 2 (DDR2); (c) detecting and reading a signal of the exosome after the contact to determine whether the agent forms a complex with DDR2 in the exosome; and (d) determining whether the signal exceeds a predetermined threshold, and when the signal exceeds the predetermined threshold, determining that the subject has the neurodegenerative disease, and wherein the threshold is a median level from subjects not having the disease.

In a twelfth aspect, the present invention provides a method for treating a neurodegenerative disease in a subject, the method comprising: (a) isolating an exosome from the subject; (b) bringing the isolated exosome into contact with an agent capable of binding to discoidin domain receptor 2 (DDR2); (c) detecting and reading a signal of the exosome after the contact to determine whether the agent forms a complex with DDR2 in the exosome; (d) determining whether the signal exceeds a predetermined threshold, and when the signal exceeds the predetermined threshold, determining that the subject has the neurodegenerative disease, and wherein the threshold is a median level from subjects not having the disease; and (e) administering a neuroprotective or neurorestorative therapy to the subject determined to have the neurodegenerative disease. In preferred embodiments, the neuroprotective or neurorestorative therapy is, for example: cholinesterase inhibitors, such as galantamine, donepezil, huperzine a, and rivastigmine; NMDA receptor antagonists, such as memantine; inflammatory factor inhibitors, such as non-steroidal anti-inflammatory drugs; glutamate inhibitors such as riluzole; and free-radical scavengers, such as edaravone.

In any one of the aspects described above, the exosome is from a bodily fluid of the subject. In some embodiments, the bodily fluid comprises one or more of peripheral blood, serum, plasma, serosal fluid, sputum, synovial fluid, aqueous humor, amniotic fluid, breast milk, semen, prostatic fluid, Cowper's fluid, female ejaculate, sweat, excreta, tears, cyst fluid, pleural fluid, ascites, pericardial fluid, chyle, bile, interstitial fluid, menses, pus, vomit, vaginal secretions, mucosal secretions, pancreatic fluid, blastocoel fluid, umbilical cord blood, urine, cerebrospinal fluid, saliva, lymph fluid, watery stools, bronchopulmonary aspirates, bronchoalveolar lavage fluid, and nasal lavage fluid. In some embodiments, the bodily fluid is serum or plasma. In some embodiments, the exosome is isolated from a sample from the subject by size exclusion chromatography, density gradient centrifugation, differential centrifugation, nanomembrane ultrafiltration, immunoabsorbent capture, affinity capture, microfluidic separation, or combinations thereof.

In any one of the aspects described above, the agent capable of binding to DDR2 comprises a protein, nucleic acid, or small molecule compound. In some embodiments, the agent capable of binding to DDR2 is an anti-DDR2 monoclonal antibody or an antigen-binding fragment thereof, or an anti-DDR2 polyclonal antibody. In some embodiments, the agent capable of binding to DDR2 is labeled with a detectable label. In some embodiments, the detectable label is selected from a fluorescent label, a chemiluminescent label, a paramagnetic label, a radioisotope label, and an enzyme label.

Additional aspects and advantages of the present invention will be described in detail in the detailed description. A person skilled in the art will be able to recognize other aspects and advantages not explicitly stated in the present disclosure from the detailed description below.

### BRIEF DESCRIPTION OF THE DRAWINGS

**FIG. 1****:** Graph of the affinity constant between the DDR2 nanobody and the extracellular domain of DDR2 antigen.
**FIG. 2****:** Graph of imaging results of ALS mouse model.
**FIG. 3****:** Graph of imaging results of AD mouse model.
**FIG. 4****:** Graph of Western blot detection results of exosomes the plasma of ALS patients.
**FIG. 5****:** Graph of flow cytometry detection results of exosomes in the plasma of ALS patients.
**FIG. 6****:** The expression of DDR2 in ALS-related experimental data.
**FIG. 7****:** The expression of DDR2 in AD-related experimental data.
**FIG. 8****:** The expression of DDR2 in AD-related experimental data.
**FIG. 9****:** Correlation analysis of DDR2 with other diagnostic genes.
**FIG. 10****:** *In-vivo* fluorescence imaging of DDR2 nanobody 1A12-ICG.
**FIG. 11****:** PET/CT imaging of AD mice.
**FIG. 12****:** PET/CT imaging of ALS mice.

### DETAILED DESCRIPTION

### Definitions

Throughout the description and claims, unless otherwise indicated in the context, the word "include" and variations such as "comprise" and "contain" will be understood to imply the inclusion of a stated integer, step or component but not the exclusion of any other integer, step or component. When used herein, the term "include" may be substituted with the term "comprise" or "contain" or sometimes with the term "have" as used herein.

In the present invention, "about" means that a value is within an acceptable error range for the particular value determined by one of ordinary skill in the art; the value will depend in part on how it is measured or determined (i.e., the limitations of the measurement system). For example, "about" may mean within 1 or more than 1 standard deviation in each practice in the art. Alternatively, "about" or "substantially comprising" may mean a range of up to 20%. Furthermore, for biological systems or processes, the terms may mean at most an order of magnitude or at most 5 times the value. Unless otherwise stated, when a particular value appears in the present application and claims, the meaning of "about" or "substantially comprising" should be assumed to be within an acceptable error range for that particular value.

As used herein, the term "subject", "patient", or "individual" refers to any subject, particularly a mammalian subject, for whom diagnosis, prognosis or treatment is desired. Mammals include humans, domestic animals, farm animals, zoo animals, sports animals, or companion animals such as dogs, cats, pigs, rabbits, rats, mice, horses, or cows. The subject referred to herein is preferably a human. In some embodiments, the subject is suffering from or susceptible to one or more disorders or conditions. The patient may exhibit one or more symptoms of a disorder or condition, or may have been diagnosed with one or more disorders or conditions. In some embodiments, the patient is receiving or has received some therapy for diagnosing and/or treating such a disease, disorder, or condition.

As used herein, the term "detection" includes any means of detection, including direct and indirect detection, quantitative and qualitative detection, and means that the presence and/or level of a specific molecule (e.g., DDR2 protein) is identified in a subject or in a sample from a subject.

As used herein, the term "diagnosis" refers to the identification or classification of a molecular or pathological condition, disease or disorder. For example, "diagnosis" may refer to the identification of a neurodegenerative disease or the identification of a particular type thereof.

According to the present invention, the term "binding" preferably relates to specific binding. "Specifically binding" means that an agent binds to a specific target more strongly than to another target. If the dissociation constant (K_{D}) for the binding of an agent to a first target is less than the dissociation constant for its binding to a second target, the agent binds to the first target more strongly than to the second target. Preferably, the dissociation constant (K_{D}) for a target to which an agent specifically binds is more than 10²-fold, 10³-fold, 10⁴-fold, 10⁵-fold, 10⁶-fold, 10⁷-fold, 10⁸-fold, 10⁹-fold, or 10¹⁰-fold lower than the dissociation constant (K_{D}) for a target to which the agent does not specifically bind.

Preferably, if an agent (e.g., a protein or polypeptide) is capable of binding to a predetermined target but not to other targets, that is, it does not have significant affinity for other targets and does not bind significantly to other targets in a standard assay, it is specific for the predetermined target. According to the present invention, if an agent is capable of binding to DDR2 but (substantially) not to other targets, it is specific for DDR2. Preferably, if the K_{D} for the binding of an agent to a predetermined target is at least 10²-fold, 10³-fold, 10⁴-fold, 10⁵-fold, 10⁶-fold, 10⁷-fold, 10⁸-fold, 10⁹-fold, or 10¹⁰-fold lower than the K_{D} for its binding to a non-specific target, the agent is specific for the target.

The binding of the agent to the target can be determined experimentally using any suitable method, which is within the scope of a person skilled in the art. Affinity can be readily determined using conventional techniques, e.g., by equilibrium dialysis; by surface plasmon resonance analysis using the general procedure outlined by the manufacturer; by radioimmunoassay using a radiolabeled target antigen; or by other methods known to the skilled person. Affinity data can be analyzed, for example, by methods known in the art. The measured affinity of a specific interaction may vary if measured under different conditions (e.g., salt concentration or pH). Therefore, measurements of affinity and other binding parameters (e.g., K_{D} or IC₅₀) are preferably performed with a standardized solution of a binder and a target and a standardized buffer.

As used herein, the term "antibody" refers to any form of antibody that exhibits the desired biological activity (e.g., inhibiting binding of a ligand to its receptor or by inhibiting ligand-induced receptor signaling). "Antibody fragment" and "antigen-binding fragment" refer to antigen-binding fragments of an antibody and antibody analogs, which typically include at least a part of the antigen-binding regions or variable regions (e.g., one or more CDRs) of the parent antibody. In some embodiments, the antibody is a monoclonal antibody. In some other embodiments, the antibody is a polyclonal antibody.

The term "monoclonal antibody" as used herein refers to an antibody obtained from a population of substantially homogeneous antibodies, that is, the individual antibodies constituting the population are identical except for possible naturally occurring mutations that may be present in minor amounts. Monoclonal antibodies are highly specific and may be directed against a single antigenic site. Furthermore, in contrast to conventional (polyclonal) antibody preparations that typically include a number of different antibodies directed against a number of different determinants (epitopes), each monoclonal antibody is only directed against a single determinant on the antigen. The modifier "monoclonal" indicates the character of the antibody obtained from a substantially homogeneous population of antibodies, and is not to be construed as requiring any specific method to prepare the antibody. For example, the monoclonal antibodies for use in the present invention may be prepared by hybridoma or recombinant DNA methods.

Monoclonal antibodies may include "chimeric" antibodies, humanized antibodies, or fully human antibodies. In some embodiments, the antibody forms part of a larger biomolecule, such as a fusion protein or an antibody-drug conjugate. An antibody fragment retains at least some of the binding specificity of the parent antibody. Generally, an antibody fragment retains at least 10% of the parent binding activity when the activity is expressed on a molar basis. Preferably, an antibody fragment retains at least 20%, 50%, 70%, 80%, 90%, 95%, or 100% or more of the binding affinity of the parent antibody for the target.

As used herein, the term "heavy chain antibody" refers to an antibody that lacks light chains and consists only of heavy chains, which comprises two constant regions (CH2 and CH3), one hinge region, and one heavy chain variable region (i.e., VHH). Examples include, but are not limited to, natural heavy chain antibodies, antibodies naturally devoid of light chains, heavy chain antibodies derived from conventional 4-chain antibodies, and engineered antibodies. Heavy chain antibodies may be antibodies from Camelidae species, for example, antibodies produced in camels, llamas, dromedaries, alpacas, and guanacos. Other species than camelidae may produce heavy chain antibodies that naturally lack light chains; such heavy chain antibodies are within the scope of the present invention.

As used herein, the term "nanobody" refers to a single domain antibody consisting of only heavy chain variable regions obtained by cloning the variable regions of a heavy chain antibody, also known as variable domain of heavy chain of heavy chain antibody (VHH) or single-domain antibody, which is the smallest functional antigen-binding fragment. Nanobodies recognize antigens with high specificity and affinity similar to IgG antibodies, but can penetrate tumor tissue better due to the smaller size (about 15 kD). Furthermore, nanobodies are resistant to extreme pH, thermal denaturation, proteolysis, solvents, and detergents. They can be expressed and produced in high yield and high solubility.

"Antibody fragment" and "antigen-binding fragment" refer to antigen-binding fragments of an antibody and antibody analogs, which typically include at least a part of the antigen-binding regions or variable regions (e.g., one or more CDRs) of the parent antibody. An antibody fragment retains at least some of the binding specificity of the parent antibody. Generally, an antibody fragment retains at least 10% of the parent binding activity when activity is expressed on a molar basis. Preferably, an antibody fragment retains at least 20%, 50%, 70%, 80%, 90%, 95%, or 100% or more of the binding affinity of the parent antibody for the target. Examples of antibody fragments include, but are not limited to: Fab, Fab', F(ab')₂, and Fv fragments; diabodies; linear antibodies; single chain antibody molecules, such as single chain variable fragment (scFv); nanobodies; domain antibodies; and multispecific antibodies formed from antibody fragments. Antibodies against DDR2 refer to antibodies that specifically bind to DDR2, including artificially designed antibodies, and any form of antibody, e.g., the antibody fragments and antigen-binding fragments as defined above.

An "equivalent variant" of an antibody or polypeptide refers to an antibody or polypeptide that has a certain degree of homology or sequence identity to the amino acid sequence of the antibody or polypeptide. In some aspects, the sequence identity is at least about 70%, 75%, 80%, 85%, 90%, 95%, 98%, or 99%. In some aspects, compared with a reference antibody or polypeptide, equivalent variants thereof have one, two, three, four, or five additions, deletions, substitutions, and combinations thereof. In some aspects, equivalent variants of an antibody or polypeptide retain the activity (e.g., epitope binding) or structure (e.g., salt bridge) of the reference sequence.

As used herein, "variant" of a sequence refers to a sequence that differs from the indicated sequence at one or more amino acid residues but retains the biological activity of the resulting molecule.

As used herein, the "% identity" between two sequences refers to a function of the number of identical positions shared by the sequences (i.e., % homology = number of identical positions / total number of positions × 100), taking into account the number of gaps and the length of each of the gaps, which need to be introduced for optimal alignment of the two sequences. The comparison of sequences and the determination of % identity between two sequences may be accomplished using a mathematical algorithm.

The term "nucleic acid" as used herein is intended to include deoxyribonucleic acid (DNA) and ribonucleic acid (RNA), e.g., genomic DNA, cDNA, mRNA, recombinantly produced and chemically synthesized molecules. The nucleic acid may be single-stranded or double-stranded. RNA includes in vitro transcribed RNA or synthetic RNA.

The terms "control", "control sample", "standard control," or "standard" refer to a sample that serves as a reference (typically a known reference) for comparison to a test sample. For example, a test sample may be taken from a patient suspected of having a given disease and compared to a sample from a patient with a known disease or from an individual known to be normal (non-diseased). Controls may also represent average values collected from a population of similar individuals (e.g., diseased patients or healthy individuals of similar medical background, same age, weight, etc.). Control values may also be obtained from the same individual, e.g., from a sample obtained earlier, prior to disease or prior to treatment. The skilled person will recognize that the controls can be designed to evaluate a number of parameters.

As used herein, the term "bodily fluid" or "bodily fluid sample" may generally refer to a fluid that is typically present in, and/or producible by, the body or body tissue of a subject or patient. For example, the bodily fluid can comprise one or more of peripheral blood, serum, plasma, serosal fluid, sputum, synovial fluid, aqueous humor, amniotic fluid, breast milk, semen, prostatic fluid, Cowper's fluid, female ejaculate, sweat, excreta, tears, cyst fluid, pleural fluid, ascites, pericardial fluid, chyle, bile, interstitial fluid, menses, pus, vomit, vaginal secretions, mucosal secretions, pancreatic fluid, blastocoel fluid, umbilical cord blood, urine, cerebrospinal fluid, saliva, lymph fluid, watery stools, bronchopulmonary aspirates, bronchoalveolar lavage fluid, and nasal lavage fluid, including fractions or fractions thereof. The bodily fluid samples may be mixed or pooled. The bodily fluid sample may be provided by removing bodily fluid from the patient, but may also be provided by using a previously isolated bodily fluid sample material. In some embodiments, the bodily fluid or bodily fluid sample used in the present invention is a serum or plasma sample.

As used herein, the term "exosome" refers to tiny membrane vesicles with a diameter of approximately 30-150 nm, which are secreted by various cells and contain specific proteins (for example, the transmembrane protein families CD63, CD81, and CD9 that are rich on the exosome membrane and are involved in exosome transportation), lipids, cytokines, or genetic materials. Exosomes can be secreted by various cells under both normal and pathological conditions. They are widely present in bodily fluids such as blood, saliva, urine, cerebrospinal fluid, and breast milk, are regarded as specifically secreted membrane vesicles and participate in intercellular communication. In some embodiments, exosomes may be isolated from a sample of bodily fluid from a subject by size exclusion chromatography, density gradient centrifugation, differential centrifugation, nanomembrane ultrafiltration, immunoabsorbent capture, affinity capture, microfluidic separation, or combinations thereof.

### Agent for detecting expression level of DDR2

In one aspect, the present invention provides an agent for detecting the expression level of discoidin domain receptor 2 (DDR2) for use in diagnosing a neurodegenerative disease in a subject.

As used herein, the term "agent for detecting the expression level of DDR2" refers to any agent known in the art that can be used to detect DDR2, for example, a targeting agent or affinity agent for DDR2, including especially agents that are capable of binding (particularly specifically binding) to DDR2 to form a chemically, physically or biologically detectable complex.

In some embodiments, the agent capable of binding to DDR2 comprises a protein, a nucleic acid, or a small molecule compound that can target one or more epitopes of DDR2 protein.

In some embodiments, the agent capable of binding to DDR2 is an anti-DDR2 monoclonal antibody or an antigen-binding fragment thereof, or an anti-DDR2 polyclonal antibody. Anti-DDR2 antibodies are available from commercial sources, e.g., GTX102526 (GeneTex), AF2538 (Novus Biologicals), or MAB2538 (R&D Systems). More DDR2 antibodies are found in https://www.antibodypedia.com/gene/4177/DDR2 (last accessed on September 1, 2022). Alternatively, anti-DDR2 antibodies can be generated *de novo* using methods known in the art. In some embodiments, the anti-DDR2 antibody is any form of antibody or antibody fragment as defined herein.

In some embodiments, the anti-DDR2 monoclonal antibody is an anti-DDR2 nanobody. In some embodiments, the anti-DDR2 nanobody comprises CDR1, CDR2, and CDR3, wherein CDR1 comprises or is a sequence set forth in SEQ ID NO: 1 or an equivalent variant thereof, CDR2 comprises or is a sequence set forth in SEQ ID NO: 2 or an equivalent variant thereof, and CDR3 comprises or is a sequence set forth in SEQ ID NO: 3 or an equivalent variant thereof, wherein the CDRs are defined according to IMGT. In some embodiments, the nanobody comprises or is a sequence set forth in SEQ ID NO: 4 or an equivalent variant thereof.

In some embodiments, the equivalent variants of CDR1, CDR2, and CDR3 refer to those having a single amino acid substitution, deletion, or insertion compared with the reference sequence.

In some embodiments, the equivalent variants of the nanobody refer to those having at least 75%, 80%, 85%, 90%, 95%, 98%, or 99% sequence identity to SEQ ID NO: 4, and having the same or equivalent CDR1, CDR2, and CDR3. In some embodiments, the CDR1, CDR2, and CDR3 are defined based on any one of definition schemes of IMGT, Kabat, Chothia, Contact, or AbM. In some embodiments, the CDR1, CDR2, and CDR3 are defined based on the IMGT definition scheme.

In some embodiments, the anti-DDR2 nanobody comprises CDR1, CDR2, and CDR3, wherein CDR1 comprises a sequence set forth in SEQ ID NO: 1, CDR2 comprises a sequence set forth in SEQ ID NO: 2, and CDR3 comprises a sequence set forth in SEQ ID NO: 3, wherein the CDRs are defined according to IMGT.

In some embodiments, the anti-DDR2 nanobody comprises CDR1, CDR2, and CDR3, wherein CDR1 is a sequence set forth in SEQ ID NO: 1, CDR2 is a sequence set forth in SEQ ID NO: 2, and CDR3 is a sequence set forth in SEQ ID NO: 3, wherein the CDRs are defined according to IMGT.

In some embodiments, the substitutions described herein are conservative substitutions. "Conservative (amino acid) substitution" refers to a substitution in which an amino acid residue is replaced with an amino acid having a similar side chain. Families of amino acid residues having similar side chains have been defined in the art, including basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), β-branched side chains (e.g., threonine, valine, and isoleucine), and aromatic side chains (e.g., tyrosine, phenylalanine, tryptophan, and histidine). Therefore, a non-essential amino acid residue in an immunoglobulin polypeptide are preferably substituted with another amino acid residue from the same side chain family. In another embodiment, the amino acid string may be substituted with a structurally similar string that differs in the order and/or composition of the side chain family members.

In some embodiments, the agent capable of binding to DDR2 is a peptide or a nucleic acid aptamer. Such an aptamer may be selected from oligonucleotide or peptide libraries by any method known in the art. Nucleic acid aptamers may be selected through systematic evolution of ligands by exponential enrichment (SELEX). Peptide aptamers may be selected using yeast or bacterial two-hybrid systems.

In some embodiments, the agent capable of binding to DDR2 is labeled with a detectable label. In some embodiments, the detectable label is selected from a fluorescent label, a chemiluminescent label, a paramagnetic label, a radioisotope label, and an enzyme label.

The selection of label depends on the means of detection. For example, fluorescent labels (such as indocyanine green (ICG), rare earth chelates (e.g., europium chelates)), fluorescein-type labels (e.g., fluorescein, fluorescein isothiocyanate, 5-carboxyfluorescein, 6-carboxyfluorescein, and dichlorotriazinylamine fluorescein), rhodamine-type labels (e.g., ALEXA568 (Invitrogen) or dansyl chloride), VIVOTAG 680 XLFLUOROCHROMETM (Perkin Elmer), phycoerythrin, 7-hydroxycoumarin, Lissamine, cyanine, phycoerythrin, Texas Red, BODIPY (Invitrogen), or analogs thereof are suitable for optical detection.

Chemiluminescent labels may also be employed (e.g., luminol, luciferase, luciferin, and aequorin). Such diagnosis and detection may also be accomplished by linking an agent capable of binding to DDR2 to detectable substances including, but not limited to: various enzymes, including but not limited to horseradish peroxidase, alkaline phosphatase, β-galactosidase, or acetylcholinesterase, or by linking the agent to prosthetic group complexes, for example, but not limited to: streptavidin/biotin and avidin/biotin.

Paramagnetic labels and radioisotope labels may also be employed, which are preferably detected using positron emission tomography (PET) or single-photon emission computed tomography (SPECT). Radiolabels include, but are not limited to, bismuth (²¹³Bi), carbon (¹¹C, ¹³C, ¹⁴C), chromium (⁵¹Cr), cobalt (⁵⁷Co, ⁶⁰Co), copper (⁶⁴Cu), dysprosium (¹⁶⁵Dy), erbium (¹⁶⁹Er), fluorine (¹⁸F), gadolinium (¹⁵³Gd, ¹⁵⁹Gd), gallium (⁶⁸Ga, ⁶⁷Ga), germanium (⁶⁸Ge), gold (¹⁹⁸Au), holmium (¹⁶⁶Ho), hydrogen (³H), indium (¹¹¹In, ¹¹²In, ¹¹³In, ¹¹⁵In), iodine (¹²¹I, ¹²³I, ¹²⁵I, ¹³¹I), iridium (¹⁹²Ir), iron (⁵⁹Fe), krypton (^{81m}Kr), lanthanum (¹⁴⁰La), lutetium (¹⁷⁷Lu), manganese (⁵⁴Mn), molybdenum (⁹⁹Mo), nitrogen (¹³N, ¹⁵N), oxygen (¹⁵O), palladium (¹⁰³Pd), phosphorus (³²P), potassium (⁴²K), praseodymium (¹⁴²Pr), promethium (¹⁴⁹Pm), rhenium (¹⁸⁶Re, ¹⁸⁸Re), rhodium (¹⁰⁵Rh), rubidium (⁸¹Rb, ⁸²Rb), ruthenium (⁸²Ru, ⁹⁷Ru), samarium (¹⁵³Sm), scandium (⁴⁷Sc), selenium (⁷⁵Se), sodium (²⁴Na), strontium (⁸⁵Sr, ⁸⁹Sr, ⁹²Sr), sulfur (³⁵S), technetium (⁹⁹Tc), thallium (²⁰¹Tl), tin (¹¹³Sn, ¹¹⁷Sn), xenon (¹³³Xe), ytterbium (¹⁶⁹Yb, ¹⁷⁵Yb, ¹⁷⁷Yb), yttrium (⁹⁰Y), and zinc (⁶⁵Zn); various positron-emitting metals for positron emission tomography and non-radioactive paramagnetic metal ions can be used, such as paramagnetic aluminum (Al) ion, barium (Ba) ion, calcium (Ca) ion, cerium (Ce) ion, dysprosium (Dy) ion, erbium (Er) ion, europium (Eu) ion, gadolinium (Gd) ion, holmium (Ho) ion, iridium (Ir) ion, lithium (Li) ion, magnesium (Mg) ion, manganese (Mn) ion, molybdenum (M) ion, neodymium (Nd) ion, osmium (Os) ion, oxygen (O) ion, palladium (Pd) ion, platinum (Pt) ion, rhodium (Rh) ion, ruthenium (Ru) ion, samarium (Sm) ion, sodium (Na) ion, strontium (Sr) ion, terbium (Tb) ion, thulium (Tm) ion, tin (Sn) ion, titanium (Ti) ion, tungsten (W) ion, and zirconium (Zi) ion, especially Co⁺², CR⁺², Cr⁺³, Cu⁺², Fe⁺², Fe⁺³, Ga⁺³, Mn⁺³, Ni⁺², Ti⁺³, V⁺, and V⁺⁴. Methods for preparing radiolabeled amino acids and related peptide derivatives are known in the art. For example, radioisotopes can be conjugated by the chloramine-T method.

In some embodiments, the present invention provides an agent for detecting the expression level of DDR2 for use in diagnosing a neurodegenerative disease in a subject, the agent for detecting the expression level of DDR2 comprising an anti-DDR2 nanobody and a detectable label linked to the nanobody, wherein the detectable label is a fluorescent label. In some embodiments, the fluorescent label is indocyanine green (ICG).

In some embodiments, the present invention provides an agent for detecting an expression level of DDR2 for use in diagnosing a neurodegenerative disease in a subject, the agent for detecting an expression level of DDR2 comprising an anti-DDR2 nanobody and a detectable label linked to the nanobody, wherein the detectable label is a radioisotope. In some embodiments, the radioisotope is ⁶⁸Ga or ⁶⁴Cu.

### Diagnosed disease

The inventors have found that neurodegenerative diseases can be diagnosed by detecting discoidin domain receptor 2 (DDR2).

As used herein, the term "neurodegenerative disease" refers to all diseases related to degenerative changes of nerve cells in the central nervous system. In particular, it includes, but is not limited to, conditions selected from the following: Alzheimer's disease, amyotrophic lateral sclerosis, Parkinson's disease, Huntington's disease, frontotemporal dementia, spinal muscular atrophy, prion disease, spinocerebellar ataxia, Friedreichs ataxia, primary lateral sclerosis, spinocerebellar atrophy, Machado-hoseph's disease, Lewy Body dementia, progressive bulbar palsy, progressive supranuclear palsy, multiple system atrophy, and the like. In some embodiments, the neurodegenerative disease is related to abnormal activation of astrocytes. In some embodiments, the neurodegenerative disease is Alzheimer's disease or amyotrophic lateral sclerosis.

### Alzheimer's disease (AD)

AD is an age-related progressive degenerative disease of the nervous system. The core symptom of AD is memory impairment accompanied by impairments in other cognitive domains, which has an impact on normal life and work. AD has an insidious onset, an irreversible course of disease, and a high rate of disability. Therefore, early diagnosis and treatment are of great significance for improving the survival rate of AD patients, especially for improving the survival quality of AD patients.

At present, the classical biomarkers for detecting early AD include amyloidβ-protein 42 (Aβ₄₂), total tau (T-tau) and phosphorylated tau (P-tau), and newly discovered neurogranins and microRNA. Generally, there are a significant increase in T-tau and P-tau in the cerebrospinal fluid (CSF) of AD patients, a significant decrease in Aβ₄₂, a significant increase in CSF neurogranin in AD patients, and a significant difference in the expression of miR-100, miR-1274a, and miR-146a in AD. Existing biomarkers fail to meet clinical needs, making the search for early AD biomarkers urgent.

Although there is no cure for AD, some FDA-approved drugs can slow the progression of the disease, such as cholinesterase inhibitors (which increase the amount of the neurotransmitter acetylcholine in the brain and promote intercellular signaling), NMDA receptor antagonists (which alter the signal transduction in brain cells). However, the efficacy of these drugs, especially for moderate to severe AD patients, is still not good enough to meet the clinical needs of the patients.

An increasing number of studies have shown that astrocytes have an impact on pathological changes of Aβ and tau proteins. Research on genetic gene bank show that more than 40 genetic loci are related to late-onset AD. Most of the related genes are expressed in astrocytes, and the astrocytes play a major role in the processes of neuroinflammation and neurodegeneration in AD. Therefore, astrocytes may be related to the pathological development and progression of AD.

### Amyotrophic lateral sclerosis (ALS)

ALS is the most common type of motor neuron disease in which both upper and lower motor neurons are affected. Pathologically, ALS patients show a reduction in the number of motor neurons in their spinal cord anterior horn, brainstem and motor cortex. Loss of motor neurons leads to muscle denervation and atrophy. Patients are characterized by a gradual loss of motor function, including muscle weakness and dysphagia, and eventually die of respiratory failure. Most ALS cases are sporadic with 5%-10% being familial cases. SOD1 is the first ALS-related gene to be discovered, and its mutations account for 20% of the familial cases. To date, more than 20 genes have been found to cause familial ALS when mutated, and mutations in genes have also been found in sporadic cases. Despite genetic heterogeneity, protein deposition in the affected region is a common hallmark of ALS.

There is currently no cure for ALS, but riluzole (a glutamate release inhibitor) shows a modest effect and can extend survival by several months. Over decades of research, there is still no consensus on the pathogenic mechanisms of ALS, which hampers the development of effective therapies. Another strategy attempts to enhance the survival of motor neurons by activating the endogenous neurotrophic factor pathways, rather than specifically blocking the pathogenic process leading to motor neuron death.

In health, astrocytes help protect and nourish the surrounding motor neurons. However, recent findings from ALS patients suggest that changes in astrocytes may contribute to the development of this disease. Researchers have found that in ALS, astrocytes lose important protective functions, particularly the ability to take up glutamate. This leads to the accumulation of glutamate, which damages the motor neurons. In another study, astrocytes with different mutations in the ALS gene were found to have different potential molecular patterns. This suggests that astrocytes acquire mutation-dependent changes during ALS.

### Kit

In one aspect, the present invention provides a kit for diagnosing a neurodegenerative disease in a subject, the kit comprising an agent for detecting the expression level of discoidin domain receptor 2 (DDR2), wherein the level of DDR2 in a sample from the subject being higher than the level of a control not having the disease indicates that the subject has the neurodegenerative disease.

In another aspect, the present invention provides a kit for diagnosing a neurodegenerative disease in a subject, the kit comprising an exosome from the subject, and an agent capable of binding to discoidin domain receptor 2 (DDR2) to detect the level of DDR2 expressed by the exosome, wherein the level of DDR2 expressed by the exosome being higher than the level of a control not having the disease indicates that the subject has the neurodegenerative disease.

In some embodiments, the agent for detecting the expression level of DDR2 comprises an agent capable of binding (especially, specifically binding) to DDR2 to form a chemically, physically, or biologically detectable complex, e.g., a protein, a nucleic acid, or a small molecule compound, particularly an anti-DDR2 monoclonal antibody or an antigen-binding fragment thereof, or an anti-DDR2 polyclonal antibody.

In some embodiments, the anti-DDR2 monoclonal antibody is an anti-DDR2 nanobody. In some embodiments, the anti-DDR2 nanobody comprises CDR1, CDR2, and CDR3, wherein CDR1 comprises or is a sequence set forth in SEQ ID NO: 1 or an equivalent variant thereof, CDR2 comprises or is a sequence set forth in SEQ ID NO: 2 or an equivalent variant thereof, and CDR3 comprises or is a sequence set forth in SEQ ID NO: 3 or an equivalent variant thereof. In some embodiments, the nanobody comprises or is a sequence set forth in SEQ ID NO: 4 or an equivalent variant thereof.

In some embodiments, the agent capable of binding to DDR2 is labeled with a detectable label. In some embodiments, the detectable label is selected from a fluorescent label, a chemiluminescent label, a paramagnetic label, a radioisotope label, and an enzyme label. In some embodiments, the label is indocyanine green (ICG). In some embodiments, the label is ⁶⁸Ga or ⁶⁴Cu.

In some embodiments, the kit further comprises DDR2 recombinant antigen controls at different concentrations to prepare a standard curve for quantitative identification.

### Detection or diagnosis method and computer-readable storage medium

In one aspect, the present invention provides use of discoidin domain receptor 2 (DDR2) as a marker for diagnosing a neurodegenerative disease in a subject.

In another aspect, the present invention provides a method for diagnosing a neurodegenerative disease in a subject *in vitro* or *ex vivo,* the method comprising detecting the presence and/or level of discoidin domain receptor 2 (DDR2) in a sample (e.g., brain tissue, cerebrospinal fluid, or exosome) from the subject.

In another aspect, the present invention provides an agent capable of binding to discoidin domain receptor 2 (DDR2) for diagnosing a neurodegenerative disease in a subject *in vivo.*

In each of the above aspects, the agent capable of binding to DDR2 is an anti-DDR2 monoclonal antibody or an antigen-binding fragment thereof, or an anti-DDR2 polyclonal antibody. In a preferred embodiment, the agent capable of binding to DDR2 is an anti-DDR2 monoclonal antibody or an antigen-binding fragment thereof.

In some embodiments, the anti-DDR2 monoclonal antibody is an anti-DDR2 nanobody. In some embodiments, the anti-DDR2 nanobody comprises CDR1, CDR2, and CDR3, wherein CDR1 comprises or is a sequence set forth in SEQ ID NO: 1 or an equivalent variant thereof, CDR2 comprises or is a sequence set forth in SEQ ID NO: 2 or an equivalent variant thereof, and CDR3 comprises or is a sequence set forth in SEQ ID NO: 3 or an equivalent variant thereof, wherein the CDRs are defined according to IMGT. In some embodiments, the nanobody comprises or is a sequence set forth in SEQ ID NO: 4 or an equivalent variant thereof.

Preferably, in each of the above aspects, wherein the agent capable of binding to DDR2 is labeled with a detectable label. In a preferred embodiment, wherein the detectable label is selected from a fluorescent label, a chemiluminescent label, a paramagnetic label, a radioisotope label, and an enzyme label. In a preferred embodiment, wherein the detectable label is selected from a fluorescent label or a chemiluminescent label. In some embodiments, the label is indocyanine green (ICG). In some embodiments, the label is ⁶⁸Ga or ⁶⁴Cu.

Preferably, in each of the above aspects, the neurodegenerative disease is selected from Alzheimer's disease, amyotrophic lateral sclerosis, Parkinson's disease, Huntington's disease, frontotemporal dementia, spinal muscular atrophy, prion disease, spinocerebellar ataxia, Friedreichs ataxia, primary lateral sclerosis, spinocerebellar atrophy, Machado-hoseph's disease, Lewy Body dementia, progressive bulbar palsy, progressive supranuclear palsy, and multiple system atrophy. In some embodiments, the neurodegenerative disease is related to abnormal activation of astrocytes. In a preferred embodiment, the neurodegenerative disease is Alzheimer's disease or amyotrophic lateral sclerosis.

Various immunoassays may be used in the diagnosis method. In some embodiments, such immunoassays comprise the use of competitive and noncompetitive detection systems such as radioimmunoassay, immunochromatography, ELISA, "sandwich" immunoassay, precipitation reaction, immunoblot analysis, gel diffusion precipitation reaction, immunodiffusion assay, agglutination assay, complement fixation assay, immunoradiometric assay, fluorescent immunoassay, etc. Both *in-vitro* and *in-vivo* assays may be used.

Generally, the level of DDR2 in the sample is compared with a reference level, wherein a deviation from the reference level indicates the presence and/or stage of the neurodegenerative disease in the subject. The reference level may be a level determined in a control sample (e.g., from a healthy tissue or subject) or a median level from healthy subjects. The presence of DDR2 and/or an increased amount of DDR2 in a sample compared with a reference level, e.g., compared with a subject not having disease, may indicate the presence or risk of occurrence of a neurodegenerative disease in the subject.

In some embodiments, DDR2 is present in a sample (e.g., brain tissue, cerebrospinal fluid, or exosome) of a subject having a neurodegenerative disease in an amount at least about 2-fold, at least about 5-fold, at least about 7.5-fold, at least about 10-fold, at least about 15-fold, or at least about 20-fold compared with the subject not having disease.

The method for diagnosis allows for quantitative and/or qualitative evaluation, e.g., absolute and/or relative measurement of the target molecule, e.g., measurement of the amount of DDR2 in the sample.

In some embodiments of the method of the present invention, determining the presence and/or amount of DDR2 in the sample comprises: (i) bringing a sample (e.g., brain tissue, cerebrospinal fluid, or exosome) into contact with an agent capable of binding to DDR2, and (ii) detecting the formation of a complex between the agent and DDR2 and/or determining the amount of the complex.

In some embodiments, the detection/diagnosis method of the present invention may be used in combination with other methods for detecting/diagnosing a neurodegenerative disease. For example, the detection/diagnosis method of the present invention may be used in combination with the detection of other biomarkers (e.g., amyloidβ-protein, tau, APOE, GFAP, AQP4, MAPT, and SOD1) of a neurodegenerative disease.

In another aspect, the present invention provides a computer-readable storage medium having stored thereon computer instructions for reading and execution by a computer, the computer instructions being executed to perform a method for diagnosing whether a subject has a neurodegenerative disease, the method comprising: (a) bringing a sample from the subject into contact with an agent capable of binding to discoidin domain receptor 2 (DDR2); (b) detecting and reading a signal of the sample after the contact to determine whether the agent forms a complex with DDR2 in the sample; and (c) determining whether the signal exceeds a predetermined threshold, and when the signal exceeds the predetermined threshold, determining that the subject has the neurodegenerative disease. In a preferred embodiment, the threshold is the median level from subjects not having disease.

In another aspect, the present invention provides a computer-readable storage medium having stored thereon computer instructions for reading and execution by a computer, the computer instructions being executed to perform a method for diagnosing whether a subject has a neurodegenerative disease, the method comprising: (a) isolating an exosome from the subject; (b) bringing the isolated exosome into contact with an agent capable of binding to discoidin domain receptor 2 (DDR2); (c) detecting and reading a signal of the exosome after the contact to determine whether the agent forms a complex with DDR2 in the exosome; and (d) determining whether the signal exceeds a predetermined threshold, and when the signal exceeds the predetermined threshold, determining that the subject has the neurodegenerative disease, and wherein the threshold is a median level from subjects not having the disease.

In another aspect, the present invention provides a method for treating a neurodegenerative disease in a subject, the method comprising: (a) bringing a sample from the subject into contact with an agent capable of binding to discoidin domain receptor 2 (DDR2); (b) detecting and reading a signal of the sample after the contact to determine whether the agent forms a complex with DDR2 in the sample; (c) determining whether the signal exceeds a predetermined threshold, and when the signal exceeds the predetermined threshold, determining that the subject has the neurodegenerative disease, and wherein the threshold is a median level from subjects not having the disease; and (d) administering a neuroprotective or neurorestorative therapy to the subject determined to have the neurodegenerative disease.

In another aspect, the present invention provides a method for treating a neurodegenerative disease in a subject, the method comprising: (a) isolating an exosome from the subject; (b) bringing the isolated exosome into contact with an agent capable of binding to discoidin domain receptor 2 (DDR2); (c) detecting and reading a signal of the exosome after the contact to determine whether the agent forms a complex with DDR2 in the exosome; (d) determining whether the signal exceeds a predetermined threshold, and when the signal exceeds the predetermined threshold, determining that the subject has the neurodegenerative disease, and wherein the threshold is a median level from subjects not having the disease; and (e) administering a neuroprotective or neurorestorative therapy to the subject determined to have the neurodegenerative disease.

In some embodiments, the agent capable of binding to DDR2 comprises a protein, a nucleic acid, or a small molecule compound. In a preferred embodiment, the agent capable of binding to DDR2 is an anti-DDR2 monoclonal antibody or an antigen-binding fragment thereof, or an anti-DDR2 polyclonal antibody. In some embodiments, the agent capable of binding to DDR2 is labeled with a detectable label. In some embodiments, the detectable label is selected from a fluorescent label, a chemiluminescent label, a paramagnetic label, a radioisotope label, and an enzyme label. In some embodiments, the label is indocyanine green (ICG). In some embodiments, the label is ⁶⁸Ga or ⁶⁴Cu.

In preferred embodiments, the neuroprotective or neurorestorative therapy is, for example: cholinesterase inhibitors, such as galantamine, donepezil, huperzine a, and rivastigmine; excitatory amino acid receptor antagonists, such as memantine; inflammatory factor inhibitors, such as non-steroidal anti-inflammatory drugs; glutamate inhibitors such as riluzole; and free-radical scavengers, such as edaravone.

In some embodiments, the predetermined threshold may be the reference level described above, wherein a deviation from the reference level indicates the presence and/or stage of the related disease in the subject. The reference level may be a level determined in a control sample (e.g., from a healthy tissue or subject) or a median level from healthy subjects. The presence of DDR2 and/or an increased amount of DDR2 in a sample compared with a reference level, e.g., compared with a subject not having disease, may indicate the presence or risk of occurrence of a neurodegenerative disease in the subject.

In some embodiments, DDR2 is present in a sample (e.g., brain tissue or exosome) of a subject having a neurodegenerative disease in an amount at least about 2-fold, at least about 5-fold, at least about 7.5-fold, at least about 10-fold, at least about 15-fold, or at least about 20-fold compared with the subject not having disease.

**Sequence Listing**

| SEQ ID NO: | Description | Sequence |
|---|---|---|
| 1 | 1A12-CDR1 | GYILSPNV |
| 2 | 1A12-CDR2 | ISWGDGRT |
| 3 | 1A12-CDR3 | AASRKGYPSTRSDPYNY |
| 4 | 1A12-VHH | EVQLVESGGGLVQPGGSLRLSCAASGYIL SPNVMGWFRQAPGKGRELVAAISWGDGR TYYPDSVEGRFTISRDNAKRMVYLQMNS LRAEDTAVYYCAASRKGYPSTRSDPYNY WGQGTQVTVSS |

### Examples

### Example 1: Antibody screening and affinity assay

### (1) Antibody screening

An anti-DDR2 heavy chain antibody was selected from alpacas immunized with the extracellular domain of human DDR2 (UniProtKB/Swiss-Prot: Q16832.2 aa 22 to aa 399). The antibody was then sequenced to confirm its VHH portion, and a nanobody, named 1A12, was obtained. The sequence of the nanobody is shown in the "Sequence Listing" section above. The nanobody was expressed and purified for further characterization and experiments.

### (2) Affinity of DDR2 nanobody for extracellular domain of DDR2 antigen

### Experimental procedures:

For the DDR2 nanobody 1A12 described above, based on the ForteBio bio-layer interferometry (BLI) platform for molecular interaction analysis, the affinity of the nanobody for the extracellular domain protein of the DDR2 antigen was analyzed and detected by determining the affinity of the antibody for the antigen by using the BLI method.

Experiment consumables: antibody: dissolved in PBS (pH 7.4); antigen: dissolved in PBS (pH 7.4); sensor: Ni-NTA; Kinetics Buffer: PBST (PBS + 0.02% Tween-20, pH 7.4); Regeneration Buffer: 10 mM Glycine-HCl, pH 1.7; Re-charged Buffer: 10 mM NiCl in H2O.

### Experimental protocol:

a. Pre-wet a probe in kinetics buffer for 10 min;
b. Baseline 1: Baseline the Biosensors in kinetics buffer for 180 s;
c. Immobilization: Dilute the His-tagged extracellular domain of antigen DDR2 to 20 µg/mL with kinetics buffer, and capture it by the sensors until the concentration reaches 4 nM (300 s);
d. Baseline 2: Baseline the Biosensors in kinetics buffer for 60 s;
e. Binding: Dilute the antibody solution to a certain concentration (from 100 nM, 2-fold dilution to 3.125 nM) with kinetics buffer, and immerse the sensor into the antibody solution for binding (600 s);
f. Dissociation: Dissociate the sensor in kinetics buffer (600 s);
g. Sensor regeneration: 10 mM Glycine-HCl, pH 1.7 for 5 s;
h. Neutralization: After sensor regeneration, neutralize the sensors in kinetics buffer for 5 s;
i. Repeat the regeneration step g and the neutralization step h: A total of 3 times (30 s);
j. Baseline 3: After sensor regeneration, add the sensors to 10 mM NiCl and keep it for 60 s.

The kinetic curves were made, and the relevant parameters were calculated. Appropriate binding and dissociation curves of several concentration gradients were selected. All the curves were fitted using the 1:1 binding model. Three curves with the best fitting degrees were selected for plotting and analysis. Finally, important parameters such as affinity value, binding constant, and dissociation constant were obtained.

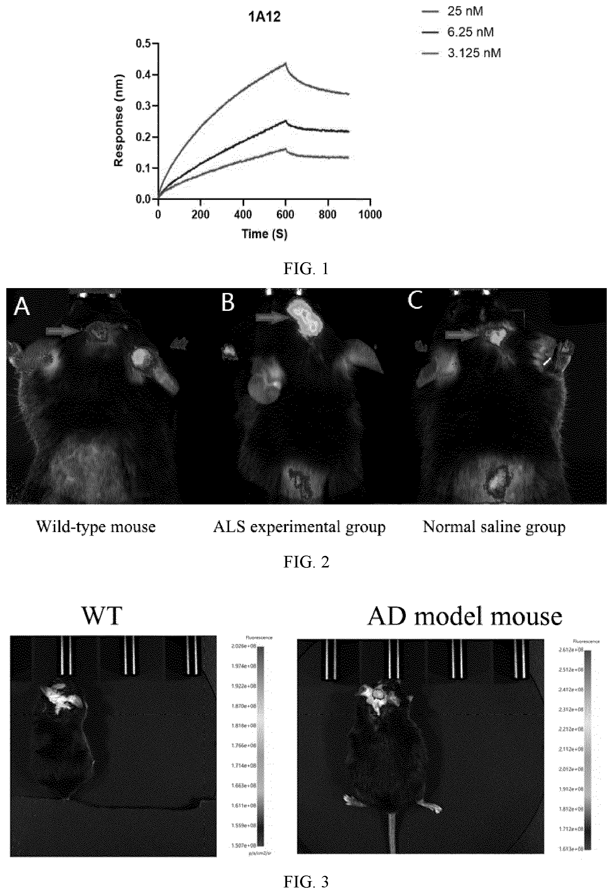

Result analysis: The analysis results of the binding of the DDR2 nanobody 1A12 to the DDR2 antigen are shown in FIG. 1 and Table 1.

**Table 1: Affinity data for nanobody 1A12**

| Nanobody No. | Affinity |
|---|---|
| 1A12 | 4.359*10^-9 |

### Example 2: In-vitro imaging results of DDR2 nanobody

The DDR2 antibody used in this example is the nanobody 1A12 (Nb-DDR2) in Example 1, and the amino acid sequence thereof is shown in SEQ ID NO: 4.

### Experimental procedures:

### (1) Labeling of the DDR2 nanobody with indocyanine green (ICG)

Nb-DDR2 was dissolved in PBS at 2 mg/mL and mixed uniformly by vortexing.

ICG-NHS was dissolved in DMSO at a concentration of 2 mM and mixed uniformly by vortexing.

500 µL of 2 mg/mL Nb-DDR2 solution was taken and placed in a 1.5 mL centrifuge tube, 18 µL of 2 mM ICG-NHS solution was added to the Nb-DDR2 solution in 9 aliquots of 2 µL each, and the mixture was mixed uniformly by vortexing for a few seconds after each addition.

The pH of the mixed solution was measured, and the pH was adjusted to 8.5-9 using a 2 M NaOH solution.

The centrifuge tube was placed on a shaker at 60 rpm for reaction at room temperature for 2 h.

The unreacted ICG-NHS was removed by centrifugation at 14,000 g for 10 min multiple times using a 0.5 mL ultrafiltration tube, the solution was replaced with 0.9% NaCl, and the protein solution was filtered through a 0.22 µm membrane and stored at 4 °C.

### (2) Imaging of amyotrophic lateral sclerosis (ALS) mouse model (lateral ventricle)

Construction of ALS mouse model: Optn-CKO mice were purchased, and Optn^{flox/+} C57BL/6J mice were constructed by Shanghai Model Organisms Center, Inc. The mice were crossed to obtain Optnf^{lox/flox}. The 3-week-old Optn^{flox/+} mice were housed separately in cages and then ear-tagged and numbered. The tails of the mice were clipped, and the genotypes of the mice were identified by PCR and agarose gel electrophoresis to screen for the Optn^{flox/flox} mice. The mice were injected with adenovirus, hAd5-Opt Cre virus, at 6.58*10¹⁰ PFU/mL and 20 µL/mouse, into the lateral ventricle at 2 months of age. After the expression of Cre enzyme, specific knockout of the OPTN gene could be performed through the loxp site. Imaging was performed 3 months after the injection of the Cre virus.

The imaging method was as follows: The DDR2 nanobody 1A12 labeled with indocyanine green (ICG) was taken and injected into the experimental mice (a wild-type mouse, an ALS model mouse) via the tail vein, and the ALS model mouse of the control group was injected with normal saline at a dose of 15 µg/mouse. Imaging was performed using an *in-vivo* imager, with the excitation light/emission light selected: 730/820 nm. The results are shown in FIG. 2.

Result analysis: The ICG-labeled DDR2 antibody could specifically recognize highly expressed DDR2 in the brain tissue of the ALS mouse, while no obvious uptake was observed in both the wild-type and normal saline controls. Therefore, by detecting the expression of DDR2, ALS can be accurately diagnosed.

### (3) Imaging of Alzheimer's disease (AD) mouse model

In this example, an APP/PS1 double transgenic mouse was used, which is a commonly used transgenic mouse for AD. The age of the mouse: 2 years. Control group: a wild-type C57 mouse.

The DDR2 nanobody 1A12 labeled with indocyanine green (ICG) was taken and injected into the experimental mice (a wild-type mouse, an AD model mouse) via the tail vein at a dose of 15 µg/mouse. Imaging was performed using an *in-vivo* imager, with the excitation light/emission light selected: 730/820 nm. The results are shown in FIG. 3.

Result analysis: the ICG-labeled DDR2 antibody could specifically recognize highly expressed DDR2 in the brain tissue of the AD mouse, while no obvious uptake was observed in the wild-type control. Therefore, by detecting the expression of DDR2, AD can be accurately diagnosed.

### Example 3: Exosome detection

### (1) Western blot (WB) detection of DDR2 in exosomes in plasma of ALS patients

The detection method was as follows:
Exosome extraction: 200 µL of plasma and 350 µL of PBS were mixed uniformly and placed in an exosome separator (EXODUS series H-300 instrument from Huixin LifeTech). The exosomes were resuspended with 400 µL of PBS. Exosome identification: 90% of the exosomes detected by the Resuntech-Nanocoulter particle sizer were in the particle size range of 30-120 nm, indicating that the extracted substances were exosomes.
A. An SDS-PAGE sample loading buffer (5×) (cat #: RM00001) was added to 10 µL of exosomes; the sample was directly loaded after boiling; an SDS-PAGE gel preparation kit (Jingcai Biotech, model number: JC-PE001) was selected.
B. The assembled electrophoresis device was placed into a tank, Marker and protein samples were loaded, and a newly prepared SDS electrophoresis buffer was poured into the tank. The voltage was set at 120 V for about 20 minutes and then changed to 240 V. The electrophoresis was stopped until the smallest molecular band of the Marker reached the bottom of the gel.
C. Membrane transfer: (preparation of membrane transfer solution: 700 mL of ultrapure water + 200 mL of methanol + 100 mL of rapid transfer solution)
   A PVDF membrane was activated with methanol. An electrophoresis gel was put into a "sandwich" device. Since the membrane transfer was from the negative electrode to the positive electrode, the gel was on a "black sandwich surface" and the membrane was on a "transparent sandwich surface". The device was then inserted into a membrane transfer tank, the membrane transfer solution (refrigerated) was poured into the electrophoresis tank, and the membrane transfer was performed at 400 mA for 35 min.
D. Blocking: the membrane was blocked with 5% milk blocking solution or 5% BSA in a shaker for 2 h.
E. Anti-hDDR2-biotin (RD, cat # BAF2538, 1:2000); incubation at 4 °C overnight.
F. The secondary antibody was streptavidin-HRP (RD, cat # DY998, 1:5000); incubation was performed for 1 h and color development was performed by ECL. Instrumental: chemiluminescent gel imaging system, model: Biolight; GELVLew 6000 Pro.

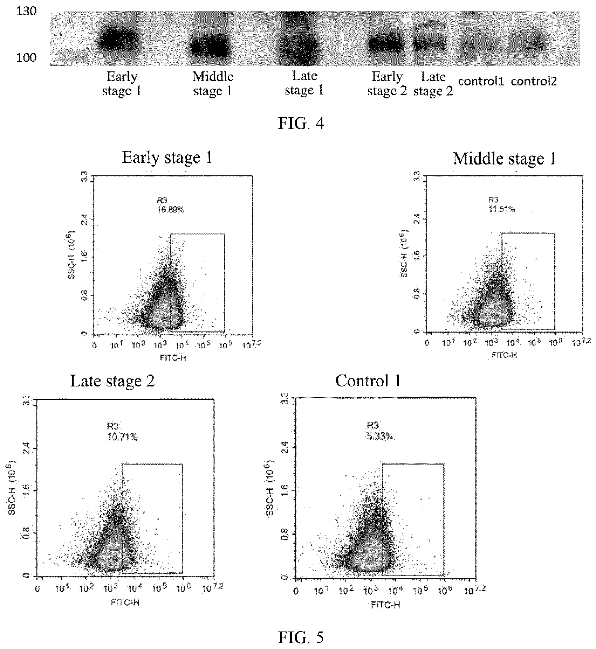

The results are shown in FIG. 4.

Result analysis: It could be seen from FIG. 4 that significant DDR2 expression could be detected in exosomes in the plasma of ALS patients at all stages compared with negative controls, indicating that ALS at all stages could be diagnosed by detecting DDR2 in exosomes.

### (2) Flow cytometry detection of DDR2 in exosomes in plasma of ALS patients

Experimental procedures for flow cytometry:
Exosome extraction: 200 µL of plasma and 350 µL of PBS were mixed uniformly and placed in an exosome separator (EXODUS series H-300 instrument from Huixin LifeTech). The exosomes were resuspended with 400 µL of PBS. Exosome identification: 90% of the exosomes detected by the Resuntech-Nanocoulter particle sizer were in the particle size range of 30-120 nm, indicating that the extracted substances were exosomes.

### Flow cytometry:

(1) The CD9 capture beads were resuspended, and 12.5 µL of the resuspended beads were aspirated and mixed with 50 µL of the sample, and the mixture was incubated at 4 °C for 18 h.
(2) After being washed twice with a wash buffer, the supernatant was discarded, and the precipitate was resuspended in 250 µL of PBS.
(3) 25 µL of the resuspension was aspirated, 1 µg of the 1A12 nanobody-FITC fluorescent antibody was added, the volume was adjusted to 100 µL with PBS, and the mixture was incubated in the dark for 1 h.
(4) After being washed twice, the precipitate was resuspended in 200 µL of PBS and detected on a flow cytometer. The results are shown in FIG. 5.

Result analysis: It could be seen from FIG. 5 that the percentage of positive DDR2 in exosomes in the plasma of ALS patients at all stages was significantly increased compared with normal people, which was at least about 2-fold higher than that of normal controls, indicating that ALS at all stages could be diagnosed by detecting DDR2 in exosomes.

In this example (including WB detection (FIG. 4) and flow cytometry (FIG. 5)), the "early", "middle", and "late" stages of ALS patients were determined based on ALS functional rating scale-revised (ALSFRS-R) scores. The scoring criteria are well known in the art and details thereof may be found, for example, in https://neurotoolkit.com/alsfrs-r/ or
https://www.outcomes-umassmed.org/ALS/alsscale.aspx (last accessed on September 19, 2022). The criteria for dividing the early, middle, and late stages: a score of 49-40 indicates the early stage, a score of 39-30 indicates the middle stage, and a score below 29 indicates the late stage.

### Example 4: Bioinformatics analysis of experimental data related to neurodegenerative diseases

### (1) Amyotrophic lateral sclerosis (ALS)

The data for analysis in this example were from the brain tissues of 13 normal donors in the Allen brain map (https://portal.brain-map.org/atlases-and-data/rnaseq/human-m1-10x) and from a total of 239,531 cells from the motor cortex tissues of the brains of 16 ALS patients from NCBI GSE174332.

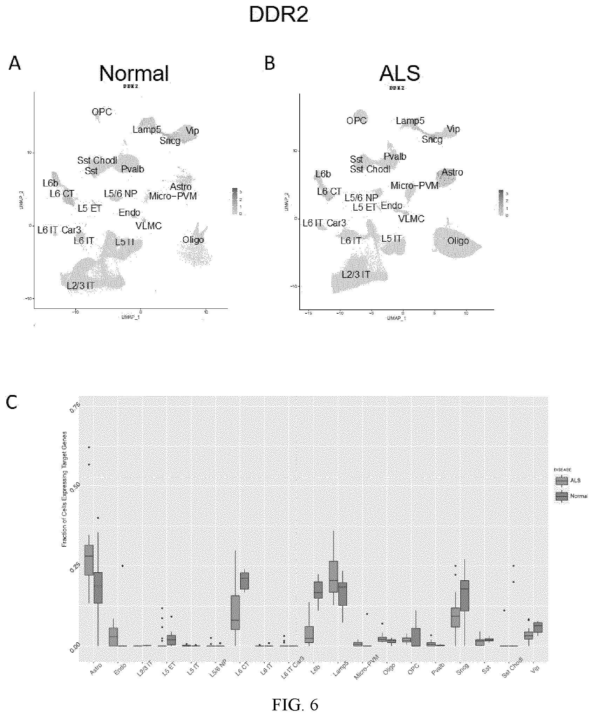

After the data were summarized, the expression of DDR2 was analyzed. The results are shown in FIG. 6. FIGs. 6A, B and C show the cell distribution and the expression level of DDR2 in normal brain tissues and brain tissues of ALS patients, and the proportion and distribution of cells expressing DDR2, respectively.

Result analysis: It could be seen from FIG. 6 that the expression of DDR2 of astrocytes (Astro) was significantly increased in ALS samples compared with normal controls, indicating that ALS could be diagnosed by detecting DDR2.

### (2) Alzheimer's disease (AD)

The original data in this example: https://www.ncbi.nlm.nih.gov/geo/query/acc.cgi?acc=GSE147528, and the data source after quality control: https://www.synapse.org/#!Synapse:syn21788402. The data consisted of 20 samples taken from the entorhinal cortex (EC) and the superior frontal gyrus (SFG) of ten AD patients. The number of cells in the entorhinal cortex: 42,528, and the number of cells in the superior frontal gyrus: 63,508.

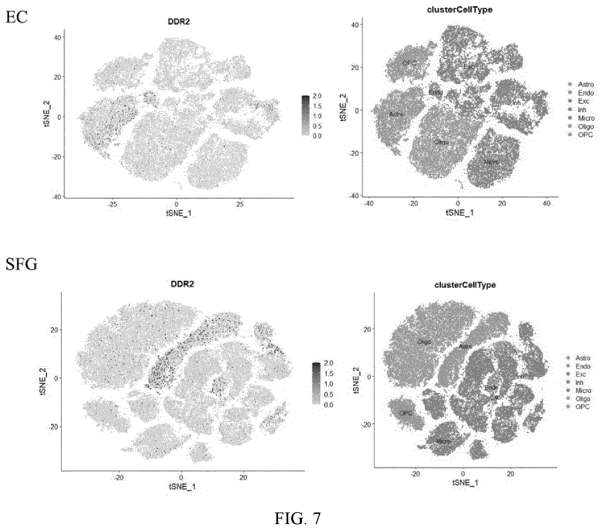

The upper and lower right panels of FIG. 7 are tSNE cell dimension reduction plots of the entorhinal cortex (EC) and the superior frontal gyrus (SFG) of ten patients, respectively, and the left panels are Feature plots of DDR2 in which each dot represents a cell, and the shade of color represents the expression level of the gene.

Result analysis: It could be seen from FIG. 7 that DDR2 was highly expressed in astrocytes in AD samples.

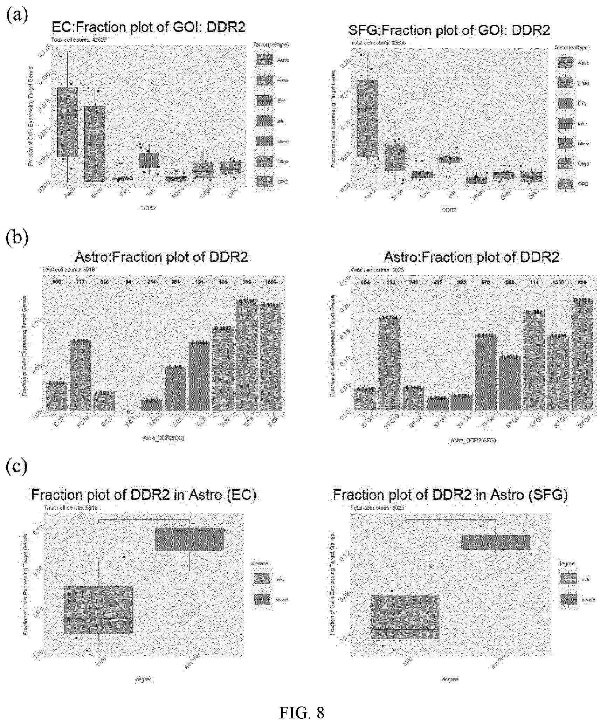

The two boxplots of FIG. 8(a) show the proportions of cells expressing DDR2 among different types of cells in the entorhinal cortex (EC) and the superior frontal gyrus (SFG), respectively, with the vertical axis indicating the proportion of cells expressing DDR2 and the horizontal axis indicating different cell types, in which each dot represents a sample.

The two histograms of FIG. 8(b) show the cell proportion of astrocytes expressing DDR2 of different samples in the entorhinal cortex (EC) and the superior frontal gyrus (SFG), respectively, with the vertical axis indicating the proportion of cells expressing DDR2 and the horizontal axis indicating different samples. The numbers above indicate the number of cells of that cell type.

Patients with braak stages 0 and 2 were classified into the mild group (mild), and patients with Braak stage 6 were classified into the severe group (severe). The two boxplots of FIG. 8(c) show the cell proportion of astrocytes expressing DDR2 in the entorhinal cortex (EC) and the superior frontal gyrus (SFG) of patients with different disease severities, with the vertical axis indicating the proportion of cells expressing DDR2 and the horizontal axis indicating different disease severities, in which each point represents a sample. Wlicox.test was used as the statistical test method.

Result analysis: It could be seen from FIG. 8 that the proportion of cells expressing DDR2 in the astrocytes of AD samples was relatively large, and a significant proportion of astrocytes expressed DDR2 in most of the samples; moreover, the proportion of astrocytes expressing DDR2 in severe AD patients was significantly increased compared with mild AD patients.

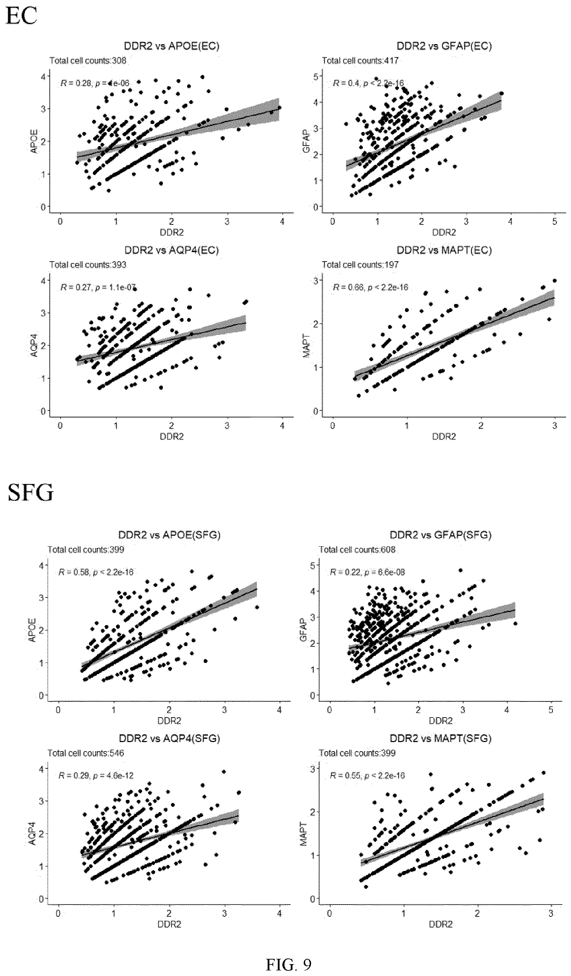

FIG. 9 shows the correlation analysis of DDR2 with the expression of APOE, GFAP, AQP4, and MAPT in astrocytes of the entorhinal cortex (EC) and the superior frontal gyrus (SFG) of ten patients. The test method was Spearman, and the cells in which both genes for the correlation analysis were expressed were selected after screening.

Result analysis: It could be seen from FIG. 9 that DDR2 had a good correlation with the existing AD detection markers.

In summary, DDR2 is significantly expressed in the samples of the AD patients and is mainly concentrated in astrocytes; moreover, as an AD diagnosis marker, DDR2 has good consistency with the existing detection markers, and can be used for diagnosing AD.

### Example 5

The ICG-labeled DDR2 nanobody 1A12 was prepared as described in Example 2.

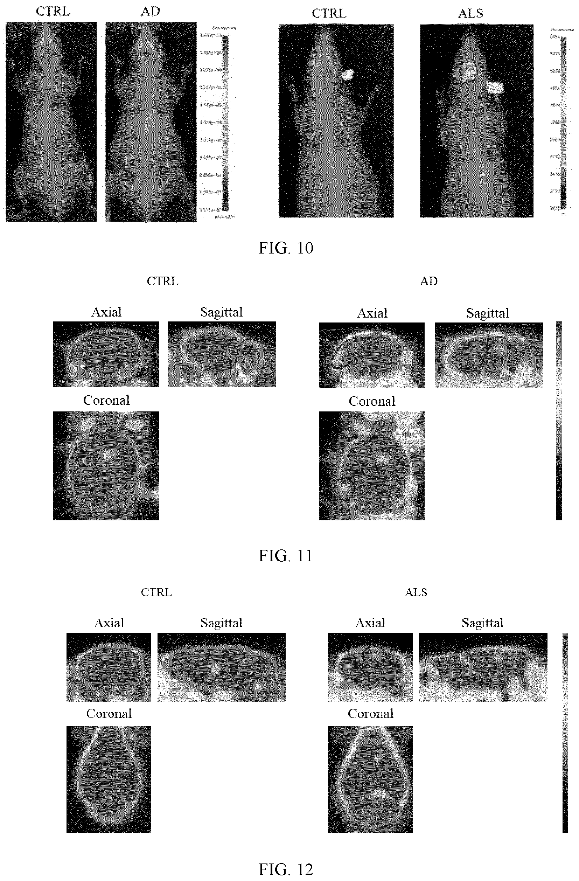

An APP/PS1 (C57BL/6) transgenic mouse (AD model, senile dementia model) was used, and a C57BL/6 mouse with the same background was used as a control mouse. The AD model mouse and the control mouse were injected with 15 µg of ICG-labeled DDR2 nanobody 1A12 probe via the tail vein. After 15 min of *in-vivo* circulation, *in-vivo* imaging was performed on the mice. The results are shown in the left panel of FIG. 10. A significant fluorescence signal could be observed in the brain of the AD model mouse compared with the control group (C57BL/6 normal mouse). The AD model mouse had developed the disease.

An SOD1 gene mutation mouse was selected as an amyotrophic lateral sclerosis (ALS) mouse model, and a C57BL/6 mouse was used as a normal control mouse. The ALS model mouse and the control mouse were injected with 15 µg of ICG-labeled DDR2 nanobody 1A12 probe via the tail vein. After 15 min of *in-vivo* circulation, *in-vivo* imaging was performed on the mice. The results are shown in the right panel of FIG. 10. A significant fluorescence signal could be observed in the brain of the ALS model mouse compared with the control group (C57BL/6 normal mouse). The ALS model mouse had developed the disease.

### Example 6: Process for labeling nanobody 1A12 with nuclides (conventional nuclides such as ⁶⁴Cu and ⁶⁸Ga)

### 1. Coupling with NOTA

(1) The antibody solution was centrifuged at 12,000 g and the supernatant was collected. The solvent of the antibody solution was replaced with 0.1 M ammonium acetate (pH = 7), and after the replacement, the concentration of the nanobody 1A12-cys was adjusted to 1.33 mg/mL. The concentration was measured before and after the replacement, the concentration after the replacement was taken as the standard.
(2) 50 mM Mal-NOTA (dissolved in DMSO) was taken such that the molar ratio of the antibody to Mal-NOTA was 1:5, and the Mal-NOTA was diluted with DMSO such that its volume was less than 10% of the volume of the reaction system (for example, if the reaction system was 1 mL, the Mal-NOTA was diluted to 80-90 µL, and mixed uniformly by shaking to ensure that the Mal-NOTA was completely dissolved in the DMSO). The Mal-NOTA solution was added to the antibody solution in portions of 10 µL each, and the mixture was mixed uniformly by shaking for tens of seconds after each addition. The pH of the solution was adjusted to 7 (the reaction solvent was 0.1 M ammonium acetate, and the pH substantially did not need to be adjusted and only required measurement and confirmation). The solution was then placed on a shaker for reaction for 2 h, with the speed of the shaker being 150 rpm.
(3) After the reaction was completed, the antibody solution was centrifuged at 12,000 g, and the supernatant was taken. The antibody solution was purified and concentrated using 0.1 M ammonium acetate, with each 100 µg of antibody concentrated to 50-80 µL, and the antibody solution was sealed with a sealing film and stored in a refrigerator at the temperature of -80 °C.

### 2. Labeling with conventional nuclides such as ⁶⁸Ga and ⁶⁴Cu

(1) ⁶⁸Ga or ⁶⁴Cu or other nuclides were taken and added to a precursor solution (the concentration of ⁶⁴Cu was as high as possible, the activity of 100 µg of precursor added was about 1.5 mCi, and the final reaction volume was not more than 150 µL), and the mixture was mixed uniformly. The pH value was adjusted to 4-5 (generally, adjustment was not needed, and the pH value was 4-5 after uniform mixing).
(2) If there was a temperature-controlled shaker, the solution was placed in the temperature-controlled shaker for reaction at 37 °C for 2 h. If there was no temperature-controlled shaker, the solution was heated at 37 °C for 2 h (with a water bath, an oven, etc.).
(3) The samples were spotted on chromatography paper and developed with a developing solvent, and the labeling rate was detected by iTLC (the developing solvent was sodium citrate).

### Example 7: Imaging of nanobody 1A12 labeled with nuclide ⁶⁴Cu

The ⁶⁴Cu-labeled Nanobody 1A12 was prepared according to the process described in Example 6.

The same AD model mouse and ALS model mouse as those in Example 5 were used and separately compared with a control normal mouse. The experimental mice described above were all injected with 200 µCi 64 Cu-labeled DDR2 nanobody 1A12 probe via the tail vein. After 45 min of *in-vivo* circulation, PET imaging data were collected, and PET/CT images were obtained after reconstruction. The uptake signal was shown in the circle.

The results are shown in FIGs. 11 and 12. The results showed that the AD model mouse and the ALS model mouse had probe uptake signals in the cerebral cortex and the motor cortex, respectively. The mouse of the normal control group did not have uptake signals.

It should be understood that although the present invention has been specifically disclosed through preferred embodiments and optional features, a person skilled in the art may make modifications, improvements, and variations to the present invention disclosed herein, and these modifications, improvements, and variations are considered to be within the scope of the present invention. The materials, methods, and examples provided herein are representative and exemplary of preferred embodiments and are not intended as limitations on the scope of the present invention.

## Claims

1. Use of an agent for detecting the expression level of discoidin domain receptor 2 (DDR2) in the preparation of a kit for diagnosing a neurodegenerative disease in a subject, wherein the level of DDR2 in a sample from the subject being higher than the level of a control not having the disease indicates that the subject has the neurodegenerative disease.

2. The use according to claim 1, wherein the agent for detecting the expression level of DDR2 comprises an agent capable of binding to DDR2 to detect the level of DDR2 in the sample.

3. The use according to claim 2, wherein the agent capable of binding to DDR2 is an anti-DDR2 monoclonal antibody or an antigen-binding fragment thereof, or an anti-DDR2 polyclonal antibody.

4. The use according to claim 3, wherein the anti-DDR2 monoclonal antibody is an anti-DDR2 nanobody.

5. The use according to claim 4, wherein the anti-DDR2 nanobody comprises CDR1, CDR2, and CDR3, wherein CDR1 comprises or is a sequence set forth in SEQ ID NO: 1 or an equivalent variant thereof, CDR2 comprises or is a sequence set forth in SEQ ID NO: 2 or an equivalent variant thereof, and CDR3 comprises or is a sequence set forth in SEQ ID NO: 3 or an equivalent variant thereof, wherein the CDRs are defined according to IMGT.

6. The use according to claim 4, wherein the nanobody comprises or is a sequence set forth in SEQ ID NO: 4 or an equivalent variant thereof.

7. The use according to claim 2, wherein the agent capable of binding to DDR2 is labeled with a detectable label.

8. The use according to claim 7, wherein the detectable label is selected from a fluorescent label, a chemiluminescent label, a paramagnetic label, a radioisotope label, and an enzyme label.

9. The use according to claim 1, wherein the neurodegenerative disease is selected from Alzheimer's disease, amyotrophic lateral sclerosis, Parkinson's disease, Huntington's disease, frontotemporal dementia, spinal muscular atrophy, prion disease, spinocerebellar ataxia, Friedreichs ataxia, primary lateral sclerosis, spinocerebellar atrophy, Machado-hoseph's disease, Lewy Body dementia, progressive bulbar palsy, progressive supranuclear palsy, and multiple system atrophy.

10. The use according to claim 1, wherein the neurodegenerative disease is Alzheimer's disease.

11. The use according to claim 1, wherein the neurodegenerative disease is amyotrophic lateral sclerosis.

12. A computer-readable storage medium having stored thereon computer instructions for reading and execution by a computer, the computer instructions being executed to perform a method for diagnosing whether a subject has a neurodegenerative disease, the method comprising:
(a) bringing a sample from the subject into contact with an agent capable of binding to discoidin domain receptor 2 (DDR2);
(b) detecting and reading a signal of the sample after the contact to determine whether the agent forms a complex with DDR2 in the sample; and
(c) determining whether the signal exceeds a predetermined threshold, and when the signal exceeds the predetermined threshold, determining that the subject has the neurodegenerative disease, and wherein the threshold is a median level from subjects not having the disease.

13. The computer-readable storage medium according to claim 12, wherein the neurodegenerative disease is Alzheimer's disease or amyotrophic lateral sclerosis.

14. Use of an exosome from a subject in the preparation of a kit for diagnosing a neurodegenerative disease in the subject, wherein the level of discoidin domain receptor 2 (DDR2) expressed by the exosome being higher than the level of a control not having the disease indicates that the subject has the neurodegenerative disease.

15. The use according to claim 14, wherein the exosome is from serum or plasma of the subject.

16. The use according to claim 14, wherein the kit further comprises an agent capable of binding to DDR2 to detect the level of DDR2 expressed by the exosome.

17. The use according to claim 14, wherein the neurodegenerative disease is Alzheimer's disease or amyotrophic lateral sclerosis.
